# EUROPEAN PATENT APPLICATION

(11) **EP 1 537 863 A1**
(43) Date of publication of application: **08.06.2005**
(21) Application number: 03792656.5
(22) Date of filing: 07.08.2003
(51) Int. Cl.: A61K 31/196, A61P 21/00

(54) **PREVENTIVE THERAPEUTIC COMPOSITION FOR MUSCULAR FATIGUE, PULLED MUSCLE AND DISEASE ATTRIBUTED THERETO**

(30) Priority: 21.08.2002 JP 2002241120
(71) Applicant: Kissei Pharmaceutical Co., Ltd., Matsumoto-shi Nagano 399-8710 (JP)
(72) Inventor: FUKUSHIMA, Kazumasa, Shinjuku-ku, Tokyo 162-0052 (JP); EBIHARA, Takahito, Nishitokyo-shi, Tokyo 202-0012 (JP)
(74) Representative: Hayes, Adrian Chetwynd
(86) International application number: PCT/JP2003/010047
(87) International publication number: WO 2004/017953

(57) **Abstract**

The present invention provides a composition for the inhibition of and promotion of recovery from muscular fatigue or muscular damage and diseases associated therewith, in particular, muscular fatigue or muscular damage caused by exercise stress and diseases associated therewith, and a composition for the prevention or treatment of myofibrosis during the restoration at the damaged part associated with or incidental to surgical injury or surgical procedure, which are **characterized by** comprising as an active ingredient *N*-(3,4-dimethoxycinnnamoyl)anthranilic acid (generic name: tranilast) or a pharmaceutically acceptable salt thereof or a pharmaceutically acceptable solvate thereof, and a method for use thereof.

## Description

### Technical Field

The present invention relates to a composition for the prevention or treatment of muscular fatigue, muscular damage and a disease associated therewith.

More particularly, the present invention relates to a composition for the inhibition of and promotion of recovery from muscular fatigue or muscular damage and a disease associated therewith, in particular, muscular fatigue or muscular damage caused by exercise stress and a disease associated therewith, and a composition for the prevention or treatment of myofibrosis during the restoration of a damaged part associated with or incidental to surgical injury or surgical procedure, which are characterized by comprising as an active ingredient *N*-(3,4-dimethoxycinnnamoyl)anthranilic acid (generic name: tranilast, hereinafter referred to as tranilast) or a pharmaceutically acceptable salt thereof or a pharmaceutically acceptable solvate thereof.

In addition, the present invention provides a method for use for the inhibition of and promotion of recovery from muscular fatigue or muscular damage and a disease associated therewith, in particular, muscular fatigue or muscular damage caused by exercise stress and a disease associated therewith, and for the prevention or treatment of myofibrosis during the restoration of a damaged part associated with or incidental to surgical injury or surgical procedure of a composition which are characterized by comprising an effective amount of tranilast, a pharmaceutically acceptable salt thereof or a pharmaceutically acceptable solvate thereof.

Furthermore, the present invention provides a method for use of a composition for the prevention or treatment of muscular fatigue or muscular damage and a disease associated therewith, and a method for use of said composition for the inhibition of and promotion of recovery from muscular fatigue or muscular damage and a disease associated therewith, which are characterized by continuously administrating or ingesting said composition for a period from at least 2 days before to 3 days or over, preferably to from 1 week to 10 days or over, after exercise stress or a surgical procedure.

### Background Art

Up to the present, in case that muscular fatigue or muscular damage and diseases associated therewith, in particular, muscular fatigue or muscular damage caused by exercise stress and diseases associated therewith occur, there mostly has been taking a rest, a light training or a rehabilitation till spontaneous recovery, and only a symptomatic treatment medicine such as antiphlogistics, analgesics or poultices has been used. Therefore, it has been desired to develop a medicine which exhibits sure therapeutic effect on and promotion of recovery in muscular fatigue, muscular damage and diseases associated therewith.

On the other hand, during the restoration of a damaged part of muscle injury such as a pulled muscle, it is said that complete recovery is histologically difficult, since the proliferation of myofibroblast predominantly progresses in comparison with the proliferation or reproduction of myoblast and, as a result, the balance between the proliferation of myoblast and the proliferation of myofibroblast is bad in the spontaneous recovery. Also, the predominant progress of the proliferation of myofibroblast compared with the proliferation of the myoblast causes the myofibrosis at the damaged part. Thus, it decreases the elasticity of muscle and is one of main causes of recurrence.

Accordingly, specially, in the field of the sports, it has been extremely desired to develop a medicine being able to inhibit the myofibrosis at the damaged part and recover said part to a regular condition as before, by controlling myofibrosis at the damaged part during the restoration associated with or incidental to surgical injury or surgical procedure and by regulating the balance between the proliferation of myofibroblast and the proliferation of myoblast.

An object of the present invention is to develop a composition for the prevention or treatment of muscular fatigue, muscular damage and diseases associated therewith, which can prevent or treat effectively muscular fatigue, muscular damage and diseases associated therewith and can inhibit the myofibrosis at the damaged part caused during the restoration thereof associated with or incidental to muscular damage such as a pulled muscle and surgical injury or surgical procedure, and recover the damaged part to a regular condition as before.

### Disclosure of the Invention

The present invention relates to
1) a composition for the prevention or treatment of muscular fatigue, muscular damage and a disease associated therewith, which is characterized by comprising as an active ingredient *N*-(3,4-dimethoxycinnnamoyl)anthranilic acid or a pharmaceutically acceptable salt thereof or a pharmaceutically acceptable solvate thereof;
2) a composition to use for the inhibition of and promotion of recovery frommuscular fatigue or muscular damage and a disease associated therewith, comprising an effective amount of *N* -(3,4-dimethoxycinnnamoyl)anthranilic acid or a pharmaceutically acceptable salt thereof or a pharmaceutically acceptable solvate thereof;
3) a composition described in the above 1) or 2) wherein the muscular fatigue or muscular damage is caused by exercise stress;
4) a composition described in the above 1) or 2) wherein the muscular damage is associated with or incidental to a surgical injury or a surgical procedure;
5) a composition described in the above 1) or 2) wherein the muscular damage is a pulled muscle;
6) a composition described in the above 1) or 2) wherein the disease is a muscle pain;
7) a composition described in the above 1) or 2) wherein the disease is myofibrosis at a damaged part caused during restoration of muscular damage;
8) a composition described in any one of the above 1) to 7), which is characterized by further comprising a pharmaceutically acceptable additive;
9) a composition described in any one of the above 1) to 7), which is characterized by further comprising an additive acceptable as food;
10) a composition described in any one of the above 1) to 9), which is a drink;
11) a composition described in any one of the above 1) to 9), which is a jerry;
12) a use of a composition for the treatment described in any one of the above 1) to 11) for the inhibition of and promotion of recovery from muscular fatigue or muscular damage and a disease associated therewith;
13) a use described in the above 12) wherein the muscular fatigue or muscular damage and the disease associated therewith are caused by exercise stress;
14) a use described in the above 12) wherein the muscular damage is associated with or incidental to a surgical injury or a surgical procedure;
15) a use described in the above 12) wherein the muscular damage is a pulled muscle;
16) a use described in the above 12) wherein the disease is a muscle pain;
17) a use described in the above 12) wherein the disease is myofibrosis at a damaged part caused during restoration of muscular damage;
18) a use described in any one of the above 12) to 17), which is characterized by continuously administrating or ingesting said composition for a period from at least 2 days before to 3 days or over after exercise stress or a surgical procedure;
19) a method for use of a composition described in any one of the above 1) to 11), which is characterized by continuously administrating or ingesting said composition for a period from at least 2 days before to 3 days or over after exercise stress or a surgical procedure;
20) a use of *N*-(3,4-dimethoxycinnnamoyl)anthranilic acid or a pharmaceutically acceptable salt thereof or a pharmaceutically acceptable solvate thereof for the manufacture of a composition described in any one of the above 1) to 11);
21) a method for the prevention or treatment of muscular fatigue or muscular damage and a disease associated therewith, which comprises administering an effective amount of N-(3,4-dimethoxycinnnamoyl)anthranilic acid or a pharmaceutically acceptable salt thereof or a pharmaceutically acceptable solvate thereof;
22) a method for the prevention or treatment described in the above 21) wherein the disease is a disease described in any one of the above 3) to 7);
23) a method for the inhibition of and promotion of recovery frommuscular fatigue or muscular damage and a disease associated therewith, which comprises administering an effective amount of N-(3,4-dimethoxycinnnamoyl)anthranilic acid or a pharmaceutically acceptable salt thereof or a pharmaceutically acceptable solvate thereof;
24) a method for the inhibition and promotion of recovery described in the above 23) wherein the disease is a disease described in any one of the above 3) to 7); and the like.

### Detailed Description of Drawings

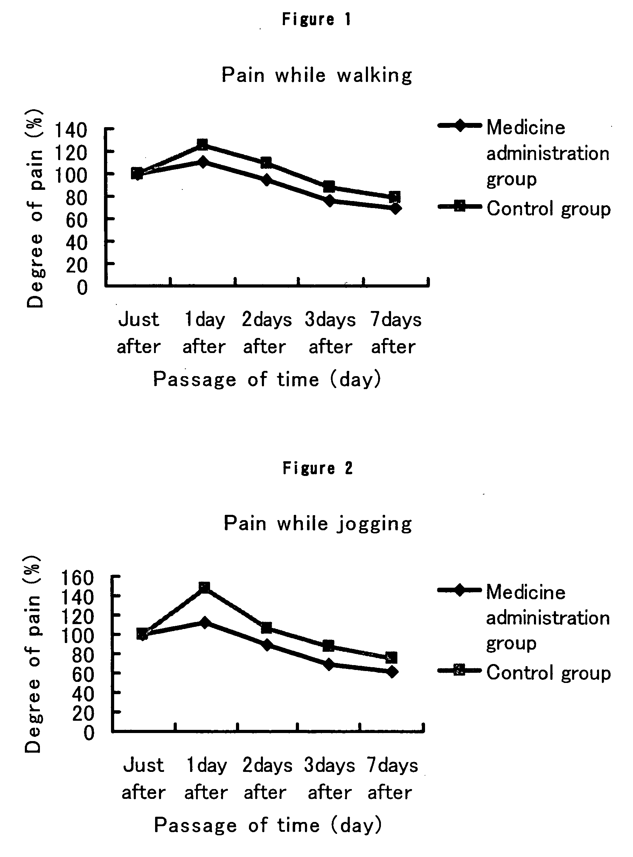

The Figure 1 is a graph showing the relative degrees of pain while walking at each time of just after, 1 day, 2 days, 3 days and 7 days after the exercise stress, respectively. A line graph of black diamond shaped point shows that of the medicine administration group and a line graph of black square point shows that of the control group. The vertical axis is the relative degree of pain (%) and the horizontal axis is the passage of time (day).

The Figure 2 is a graph showing the relative degree of pain while jogging at each time of just after, 1 day, 2 days, 3 days and 7 days after the exercise stress, respectively. A line graph of black diamond shaped point shows that of the medicine administration group and a line graph of black square point shows that of the control group. The vertical axis is the relative degree of pain (%) and the horizontal axis is the passage of time (day).

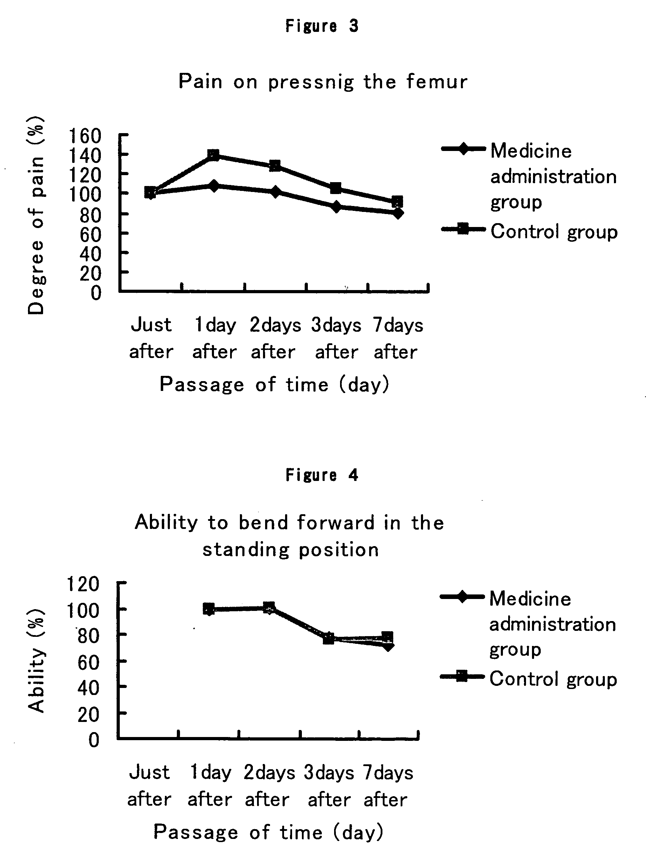

The Figure 3 is a graph showing the relative degree of pain on pressing the femur at each time of just after, 1 day, 2 days, 3 days and 7 days after the exercise stress, respectively. A line graph of black diamond shaped point shows that of the medicine administration group and a line graph of black square point shows that of the control group. The vertical axis is the relative degree of pain (%) and the horizontal axis is the passage of time (day).

The Figure 4 is a graph showing the relative ability to bend forward in the standing position at each time of just after, 1 day, 2 days, 3 days and 7 days after the exercise stress, respectively. A line graph of black diamond shaped point shows that of the medicine administration group and a line graph of black square point shows that of the control group. The vertical axis is the relative ability (%) and the horizontal axis is the passage of time (day).

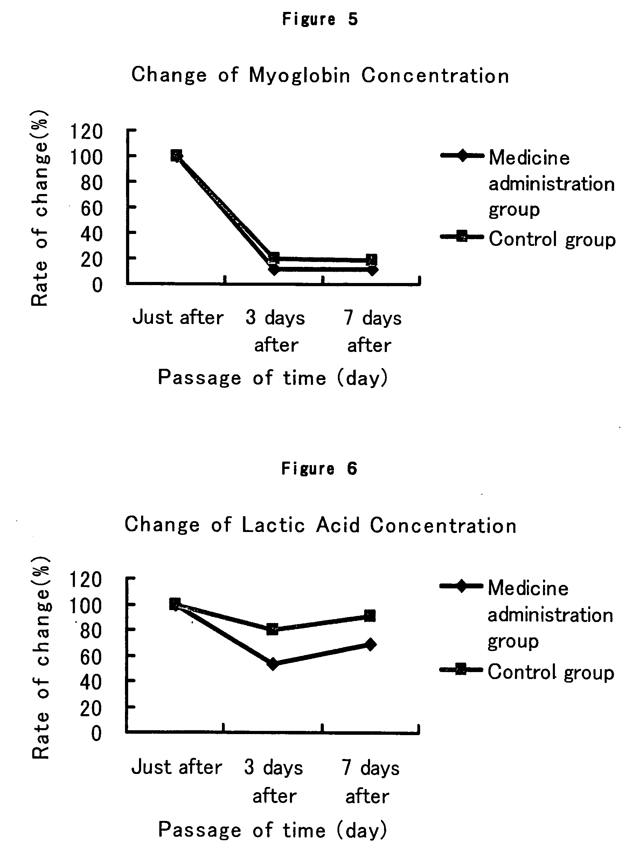

The Figure 5 is a graph showing a change of blood myoglobin concentration at each time of just after, 3 days and 7 days after the exercise stress, respectively. A line graph of black diamond shaped point shows that of the medicine administration group and a line graph of black square point shows that of the control group. The vertical axis is the rate of the change (%) and the horizontal axis is the passage of time (day).

The Figure 6 is a graph shows a change of blood lactic acid concentration at each time of just after, 3 days and 7 days after the exercise stress, respectively. A line graph of black diamond shaped point shows that of the medicine administration group and a line graph of black square point shows that of the control group. The vertical axis is the rate of the change (%) and the horizontal axis is the passage of time (day).

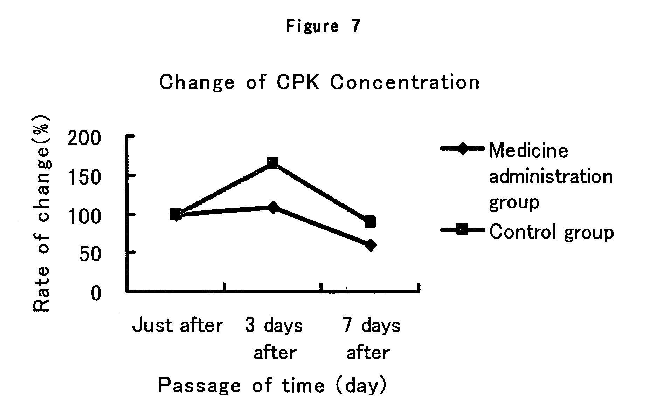

The Figure 7 is a graph showing a change of blood CPK concentration at each time of just after, 3 days and 7 days after the exercise stress, respectively. A line graph of black diamond shaped point shows that of the medicine administration group and a line graph of black square point shows that of the control group. The vertical axis is the rate of the change (%) and the horizontal axis is the passage of time (day).

### Best Mode for Carrying Out the Invention

The present inventors studied earnestly to develop a composition for the prevention or treatment of muscular fatigue, muscular damage and diseases associated therewith, which can solve the aforementioned objects. As a result, the present inventors found out that tranilast, which has inhibitory effects on release of chemical mediators, excessive collagen accumulation, etc., and is used as an agent for the prevention or treatment of allergic diseases, keloid or hypertrophic scar, etc. , exhibits an inhibitory effect on and promotion effect on recovery from muscular fatigue caused by exercise stress and also inhibits the myofibrosis at a damaged part that occurs during the restoration whereof associated with or incidental to surgical injury or surgical procedures, and moreover, can recover the damaged part to a regular condition as before, by controlling the proliferation of the myofibroblast and by regulating the balance between the proliferation of the myofibroblast and the proliferation of the myoblast. And thereby, the present invention has been completed.

That is, the present inventors studied a muscular fatigue test and a blood test on exercise stress of 15 km running within a limited time of 2 hours in 20 healthy subjects divided into 2 groups of 10 subjects each. To the subjects in one group were administered tranilast 100 mg 3 times a day for 1 week after the exercise, while to the subjects in the other group was not administered tranilast. As a result, it was surprisingly found that tranilast administration group exhibited a significantly lower degree of muscular fatigue and faster and better recovery compared with no administration group. The present invention is based on those findings.

Tranilast or a pharmaceutically acceptable salt thereof exhibits an inhibitory effect on release of chemical mediators caused by allergic reactions and has been already and extensively used as a composition for the prevention or treatment of allergic diseases such as allergic bronchitis, asthma, atopic dermatitis and allergic conjunctivitis. It is confirmed that it also exhibits inhibitory effects on excessive collagen accumulation, restenosis after percutaneous transluminal coronary angioplasty (PTCA) etc., and has been already and extensively used as a composition for the prevention or treatment of keloid or hypertrophic scar.

Furthermore, it is reported that tranilast exhibits an inhibitory effect on sclerosis of heart by the inhibition or involution of heart hypertrophy in an experimental animal model using spontaneous hypertension rats (SHR), and therefore, is useful as an agent for the prevention or treatment of hypertrophy of heart (Japanese Patent Publication No. Hei 7-277966). It is also reported that it exhibits an inhibitory effect on atherogenesis in WHHL rabbits, which is an experimental animal of Familial hypercholesterolemia model, and is useful as an agent for the treatment of atherosclerosis (Japanese Patent Publication No. Hei 9-227371). It is further reported that tranilast exhibits an inhibitory effect on release of cytokines such as transforming growth factor (TGF)-beta 1 from human monocyte macrophages and interleukin (IL)-1 beta, and prostaglandin (PG) E2 (Japanese Journal of Pharmacology, vol. 60, pp. 85-90, 1992).

However, up to now, it has been neither reported nor suggested that tranilast exhibits an inhibitory effect on and promotion effect on recovery from muscular fatigue caused by exercise stress and is useful as an agent for the prevention or treatment of muscular fatigue, muscular damage and diseases associated therewith.

The present inventors took notice of various activities of tranilast and presumed that tranilast, which exhibits inhibitory effects on excessive collagen accumulation and on restenosis after percutaneous transluminal coronary angioplasty (PTCA), might act effectively in treating diseases associated with muscular fatigue. Then, the present inventors earnestly examined the effect of tranilast on muscular fatigue, muscular damage caused by exercise stress and diseases associated therewith, and thus found out that tranilast exhibits an inhibitory effect on and promotion effect on recovery from muscular fatigue and so it is prospective as a composition for the prevention or treatment of muscular fatigue, muscular damage and diseases associated therewith.

That is, the present inventors set 20 healthy subjects an exercise stress of 15 km running within a limited time of 2 hours. The 20 subjects were divided into 2 groups consisting of one group of subjects with odd-numbered order of arrival who were administered tranilast, and the other group of subjects with even-numbered order of arrival who were not administered. To the subjects of tranilast administration group were administered 100 mg of tranilast three times a day after each meal for 1 week since after the evening meal of the day of the exercise stress. And degrees of muscle damage were tested by measuring pains while walking, while jogging and on pressing the femur and an ability to bend forward in the standing position at each time of 1 day, 2 days, 3 days and 7 days (the last administration day) after the exercise stress, respectively. Furthermore, bloods of the subjects were collected just before, just after, 3 days and 7 days after the exercise stress, respectively, and the blood component analyzing tests were performed.

As the results, tranilast administration group surprisingly exhibited an extremely effective inhibitory activity on and promoting activity on recovery from muscular fatigue and muscular damage caused by the exercise stress. That is, it is confirmed that tranilast administration group exhibits a remarkable lowering and alleviating effect on pains compared with no administration group in the tests of a pain while usual walking (hereinafter referred to as pain while walking), a pain while jogging (hereinafter referred to as pain while jogging) and a pain on pressing the femur (hereinafter referred to as pain on pressing the femur) in relative degrees of pains to the pains just after the exercise stress set as 100%. A pain of muscular fatigue caused by exercise stress occurs usually strongly 1 day or 2 days after of the exercise stress. The present tranilast administration group exhibited particularly lower values compared with the control group at 1 day after the exercise stress in relative degrees of pains to the pains just after the exercise stress set as 100 %. Furthermore, in tranilast administration group the values were significantly lowered at 2 days, 3 days and 7 days after the exercise stress compared with the control group, in a similar differential to that of 1 day thereafter.

And an ability to bend forward in the standing position to the limit (hereinafter referred to as ability to bend forward in the standing position) was also measured and compared between the tranilast administration group and the control group. It is considered that in evaluation of an ability to bend forward in the standing position, a degree of muscle fatigue does not appear clearly because it is different from the aforementioned pains and depends on individual's flexibility of body or athletic faculty. However, tranilast administration group exhibited significantly higher ability compared with the control group at 7 days thereafter.

These results demonstrate that tranilast has an inhibitory effect on and promoting effect on recovery from muscular fatigue.

Furthermore, in the blood test, in comparison with the control group, tranilast administration group exhibited significantly lower blood concentrations of myoglobin, which is considered to be produced by destruction of myioblast, and of lactic acid, which is an indicator of muscular fatigue, in relative degrees of changes of blood concentrations which is converted into relative values (%) to the values at just after the exercise stress set as 100%. And, it seems that the rate of decrease reached the plateau at 3 days after the exercise stress because the rate of decrease at 7 days thereafter showed only little changes from that at 3 days thereafter.

Creatinine phosphokinase (CPK, hereinafter referred to as CPK), which is considered as an indicator of destruction of myioblast similar to myoglobin, usually appears in blood slowly after a destruction of myioblast. Then, in tranilast administration group, the increase in the relative degree of change of blood concentration at 3 days after exercise stress, which is converted into a value relative to the value just after the exercise stress set as 100%, was only about 110 % compared to 160% or more in the control group. And furthermore, the decrease in the relative degree of change at 7 days thereafter in tranilast administration group was about 60% compared to only about 90% in the control group.

These results demonstrate that tranilast also has an inhibitory effect on muscular damage accompanying with a promoting effect on recovery from muscular damage.

On the other hand, it is expected that tranilast inhibits excessive proliferation of myofibloblast caused during the recovery from muscular damage because it has inhibitory effects on excessive collagen accumulation and on restenosis after percutaneous transluminal coronary angioplasty (PTCA). Therefore, it is also expected that tranilast can inhibit the myofibrosis at the damaged part and recover a damaged part to a regular condition as before, by controlling the proliferation of myofibroblast at the damaged part which occurs during the restoration whereof associated with or incidental to muscular damage such as a pulled muscle and surgical injury or surgical procedures and by regulating the balance between the proliferation of myofibroblast and the proliferation of myoblast.

Thus, tranilast exhibits a remarkably inhibitory effect on muscular fatigue and a remarkably promoting effect on recovery from muscular fatigue and also exhibits an inhibitory effect on muscular damage accompanying with a promoting effect on recovery from muscular damage. Furthermore, it is expected that tranilast can recover a damaged part to a regular condition as before, by controlling the proliferation of myofibroblast during the restoration of diseases associated with muscular damage and by regulating the balance between the proliferation of myofibroblast and the proliferation of myoblast. Accordingly, an extremely effective and useful composition for the prevention or treatment of muscular fatigue or muscular damage and diseases associated therewith can be formulated by comprising as an active ingredient tranilast or a pharmaceutically acceptable salt thereof or a pharmaceutically acceptable solvate thereof.

Furthermore, a treatment for recovering from muscular fatigue, muscular damage and diseases associated therewith can be performed in a short period effectively by using the present composition, instead of the existing treatments, that is, merely waiting a spontaneous recovery with taking a rest, a light training or a rehabilitation and using antiphlogistics, analgesics, poultices or the like as a symptomatic treatment medicine. And also, muscular fatigue, muscular damage and diseases associated therewith can be effectively inhibited by using the present composition preventively. Furthermore, although it was considered to be difficult to recover with a spontaneous recovery from muscular damage such as a pulled muscle, the composition of the present invention can be expected to recover a damaged part to a regular condition as before by controlling the proliferation of myofibroblast and by regulating the balance between the proliferation of myofibroblast and the proliferation of myoblast.

When the present composition is employed practically, it can be administered orally or parenterally in the form of a pharmaceutical composition as various dosage forms or can be taken alone or in combination with the other foods in the form of an edible composition. As dosage forms of the pharmaceutical compositions, oral pharmaceutical compositions such as powders, granules, tablets, capsules, dry syrups, oral liquid preparations or the like and parenteral pharmaceutical compositions such as injections, suppositories, plasters or the like can be illustrated. And as edible compositions, liquid foods such as a drink, a gel formed food such as a jelly, a pudding or the like, and powdered foods which can be taken suitably by liquefying or by admixing with the other foods can be illustrated.

Of these compositions, the pharmaceutical compositions can be prepared by suitably admixing with appropriate pharmaceutically acceptable additives such as diluents, disintegrating agents, binders, glidants and the like in the pharmaceutically usual way, and formulating in accordance with conventional methods.

For example, powders can be formulated by, if desired, admixing well an active ingredient with appropriate diluents, binders and the like.

Tablets can be formulated by, if desired, admixing an active ingredient with appropriate diluents, disintegrating agents, binders, glidants and the like, and compressing the mixture in accordance with conventional methods. The tablets, further if desired, can be suitably coated to provide film-coated tablets, sugar-coated tablets, enteric-coated tablets and the like.

Capsules can be formulated by, if desired, admixing well an active ingredient with appropriate diluents, glidants and the like, or formulating granules or fine-powders in accordance with conventional methods, and then filling the compositions in appropriate capsules.

The edible composition can be prepared by suitably admixing with food additives such as sweeteners, corrigents, flavors, coloring agents and the like, and processing in accordance with conventional methods. These edible compositions can be taken alone or in combination with the other foods.

In the present compositions, at least one member selected from a group of analgesics and antiphlogistics can be admixed as the other active ingredients than tranilast or a pharmaceutically acceptable salt thereof or a pharmaceutically acceptable solvate thereof. Furthermore, various vitamins can be admixed thereto.

The compositions of the present invention exhibit an inhibitory effect on and a promoting effect on recovery from muscular fatigue and muscular damage, and have a different mechanism from a medicine such as antiphlogistics, analgesics or poultices, which is used as a symptomatic treatment medicine in the past. And they do not cause an interaction together with each others, and therefore, the compositions of the present invention can be employed in combination with these antiphlogistics, analgesics or poultices.

When the present composition is employed practically, the dosage of an active ingredient is appropriately decided depending on the sex, body weight and degree of symptoms of each patient, and tranilast or a pharmaceutically acceptable salt thereof or a pharmaceutically acceptable solvate thereof as the active ingredient can be administered approximately within the range of from 100 to 1000 mg, preferably 300 to 600 mg, per day per adult human in the case of oral administration in terms of volumes of tranilast.

When tranilast is employed for the treatment for allergic diseases, especially for allergic asthma or pollinosis, it is mainly administered preventively in a manner of in advance of the developing of the symptoms and continuously. In the development process of this agent for the prevention or treatment of allergic diseases, it was confirmed in the pharmacokinetics study of tranilast that in case of repeated administration of 300 mg a day, tranilast concentration in blood reached to the maximum at 2 days after the administration. On the other hand, as stated above, the present inventors confirmed that tranilast exhibits certain satisfactory inhibitory effect on and promoting effect on recovery from muscular fatigue and muscular damage at 3 days after the administration. Therefore, when the present composition is employed for the prevention or treatment of muscular fatigue, muscular damage and diseases associated therewith, it is preferable to administer said composition to a patient continuously from at least 2 days before exercise stress or a surgical procedure to 3 days or over after exercise stress or a surgical procedure or after the last exercise stress in the case that exercise stress continues in a long term, more preferably, to continuously 1 week to 10 days or over after exercise stress or surgical procedure.

### Examples

The present invention is further illustrated in more detail by way of the following Examples and Formulation Examples. However, the present invention is not limited thereto.

### Example 1

To 20 healthy subjects (14 males and 6 females), was given an exercise stress of 15 km running within a limited time of 2 hours. The 20 subjects who run the whole distance within the limited time were divided into 2 groups by the arrival order, that is, one group consisting of 10 subjects of odd-numbered order of arrival (8 male subjects and 2 female subjects) and the other group consisting of 10 subjects of even-numbered order of arrival (6 male subjects and 4 female subjects). To the group of odd numbers was administered tranilast (hereinafter referred to as Medicine administration group) and to the group of even numbers was not administered (hereinafter referred to as Control group).

To the subjects in Medicine administration group was administered tranilast in a dosage of 100 mg after each meal 3 times a day for a period from the evening meal of the day of the exercise stress to after the lunch of 7 days after the exercise stress, and while to the subjects in Control group was not administered anything.

And, an effect of tranilast on muscular fatigue and muscular damage was evaluated by measuring muscular fatigue and analyzing blood components in each group.

### Tests and Results

### (1) Muscular fatigue test

In each group, a pain while walking, a pain while jogging and a pain on pressing the femur were measured and expressed numerically by using a score with a visual analogue scale method (hereinafter referred to as VAS score) at a time of just after, 1 day, 2 days, 3 days and 7 days (the last date of administration of medicine) after the exercise stress. And, values of relative degrees of pains (%) which are converted into relative values to the pains just after the exercise stress set as 100% were calculated.

As shown in Figures 1-3, the Medicine administration group exhibited particularly lower value compared with the control group in all pain tests at 1 day after the exercise stress, and furthermore, also exhibited lower value compared with the control group at 2 days, 3 days and 7 days after the exercise stress in a similar differential to that of 1 day thereafter.

Furthermore, with regard to an athletic ability, an ability to bend forward in the standing position was measured and a value of relative degree of the ability (%) which is converted into a relative value to the value of ability just after the exercise stress set as 100 % was calculated in according to that aforementioned.

As shown in Figure 4, the Medicine administration group exhibited similar values compared with the control group at 1 day, 2 days and 3 days after the exercise stress, and the Medicine administration group, however, exhibited significantly higher ability compared with Control group at 7 days after the exercise stress.

### (2) Analyzing test of blood components

In each group, bloods were collected at a time of just before, just after, 3 days and 7 days after the exercise stress. And, blood components considered relevant to muscular fatigue and muscular damage (myoglobin, lactic acid and CPK) were analyzed, and relative degrees of changes of blood concentrations which are converted into relative values to the concentrations just after the exercise stress set as 100% were calculated.

As shown in Figures 5-7 , the Medicine administration group exhibited significantly lower values compared with Control group in the blood concentrations of myoglobin and lactic acid. Additionally, it seems that the rate of decrease reached the plateau at 3 days after the exercise stress because the rate of decrease at 7 days thereafter has little changes from that at 3 days thereafter. And, the blood concentration of CPK was increased at 3 days after the exercise stress compared to just after the exercise stress, while the Medicine administration group significantly inhibited the increase of the blood concentration of CPK, compared with the control group, and furthermore, also exhibited significantly lower value compared with the control group at 7 days after the exercise stress.

### Formulation Examples

Various pharmaceutical compositions are formulated by the following formulations. However, the kinds of dosage forms and formulations of the present invention are not limited thereto.

### (1) Powders (Ten times diluted powders)

With 900 g of lactose was admixed well 100 g of tranilast to formulate powders of 1,000 g containing 100 mg of tranilast in 1 g of powders.

### (2) Powders (Two times diluted powders)

With 500 g of lactose was admixed well 500 g of tranilast to formulate powders of 1,000 g containing 500 mg of tranilast in 1 g of powders.

### (3) Tablets

With 50 g of lactose, 40 g of 6% HPC lactose, 6 g of starch and 4 g of talc was admixed well 100 g of tranilast, and the mixture was compressed to produce 1,000 tablets containing 100 mg of tranilast in 1 tablet.

### (4) Capsules

With 90 g of lactose, 6 g of starch and 4 g of calcium stearate was admixed well 100 g of tranilast, and the mixture was filled equally in hard capsules to produce 1,000 capsules containing 100 mg of tranilast in 1 capsule.

### Industrial Applicability

As stated above, tranilast exhibits a remarkably inhibitory effect on muscular fatigue and a remarkably promoting effect on recovery from muscular fatigue, and further exhibits an inhibitory effect on muscular damage with a promoting effect on recovery from muscular damage. Furthermore, it is expected that tranilast can recover a damaged part to a regular condition as before, by controlling the proliferation of myofibroblast which occurs during the restoration of muscular damage and by regulating the balance between the proliferation of myofibroblast and the proliferation of myoblast.

Accordingly, an extremely effective and useful composition for the prevention or treatment of muscular fatigue, muscular damage and diseases associated therewith can be manufactured by comprising as an active ingredient tranilast or a pharmaceutically acceptable salt thereof or a pharmaceutically acceptable solvate thereof.

Furthermore, a treatment for recovering from muscular fatigue, muscular damage and diseases associated therewith can be performed in a short period effectively by using the present composition, instead of the existing treatments, that is, merely waiting a spontaneous recovery with taking a rest, a light training or a rehabilitation and using antiphlogistics, analgesics, poultices or the like as a symptomatic treatment medicine. And also, muscular fatigue, muscular damage and diseases associated therewith can be effectively inhibited by using the present composition preventively.

## Claims

1. A composition for the prevention or treatment of muscular fatigue, muscular damage and a disease associated therewith, which is **characterized by** comprising as an active ingredient *N*-(3,4-dimethoxycinnnamoyl)anthranilic acid or a pharmaceutically acceptable salt thereof or a pharmaceutically acceptable solvate thereof.

2. A composition to use for the inhibition of and promotion of recovery frommuscular fatigue or muscular damage and a disease associated therewith, comprising an effective amount of *N*-(3,4-dimethoxycinnnamoyl)anthranilic acid or a pharmaceutically acceptable salt thereof or a pharmaceutically acceptable solvate thereof.

3. A composition as claimed in claim 1 or claim 2 wherein the muscular fatigue or muscular damage is caused by exercise stress.

4. A composition as claimed in claim 1 or claim 2 wherein the muscular damage is associated with or incidental to a surgical injury or a surgical procedure.

5. A composition as claimed in claim 1 or claim 2 wherein the muscular damage is a pulled muscle.

6. A composition as claimed in claim 1 or claim 2 wherein the disease is a muscle pain.

7. A composition as claimed in claim 1 or claim 2 wherein the disease is myofibrosis at a damaged part caused during restoration of muscular damage.

8. A composition as claimed in any one of claims 1-7, which is **characterized by** further comprising a pharmaceutically acceptable additive.

9. A composition as claimed in any one of claims 1-7, which is **characterized by** further comprising an additive acceptable as food.

10. A composition as claimed in any one of claims 1-9, which is a drink.

11. A composition as claimed in any one of claims 1-9, which is a jerry.

12. A use of a composition for treatment as claimed in any one of claims 1-11 for the inhibition of and promotion of recovery frommuscular fatigue or muscular damage and a disease associated therewith.

13. A use as claimed in claim 12 wherein the muscular fatigue or muscular damage and the disease associated therewith are caused by exercise stress.

14. A use as claimed in claim 12 wherein the muscular damage is associated with or incidental to a surgical injury or a surgical procedure.

15. A use as claimed in claim 12 wherein the muscular damage is a pulled muscle.

16. A use as claimed in claim 12 wherein the disease is muscle pain.

17. A use as claimed in claim 12 wherein the disease is myofibrosis at a damaged part caused during restoration of muscular damage.

18. A use as claimed in any one of claims 12-17, which is **characterized by** continuously administrating or ingesting said composition for a period from at least 2 days before to 3 days or over after exercise stress or a surgical procedure.

19. A method for use of a composition as claimed in any one of claims 1-11, which is **characterized by** continuously administrating or ingesting said composition for a period from at least 2 days before to 3 days or over after exercise stress or a surgical procedure.

20. A use of *N*-(3,4-dimethoxycinnnamoyl)anthranilic acid or a pharmaceutically acceptable salt thereof or a pharmaceutically acceptable solvate thereof for the manufacture of a composition as claimed in any one of claims 1-11.

21. A method for the prevention or treatment of muscular fatigue or muscular damage and a disease associated therewith, which comprises administering an effective amount of *N*-(3,4-dimethoxycinnnamoyl)anthranilic acid or a pharmaceutically acceptable salt thereof or a pharmaceutically acceptable solvate thereof.

22. A method for the prevention or treatment as claimed in claim 21 wherein the disease is a disease of any one of claims 3-7.

23. A method for the inhibition of and promotion of recovery from muscular fatigue or muscular damage and a disease associated therewith, which comprises administering an effective amount of *N*-(3,4-dimethoxycinnnamoyl)anthranilic acid or a pharmaceutically acceptable salt thereof or a pharmaceutically acceptable solvate thereof.

24. A method for the inhibition and promotion of recovery as claimed in claim 23 wherein the disease is a disease of any one of claims 3-7.
